(19) Europäisches Patentamt / European Patent Office / Office européen des brevets

(11) **EP 3 164 144 B1**

(12) **FASCICULE DE BREVET EUROPEEN**

(45) Date de publication et mention de la délivrance du brevet:
**28.07.2021 Bulletin 2021/30**

(21) Numéro de dépôt: **15722225.8**

(22) Date de dépôt: **15.05.2015**

(51) Int Cl.:
*A61K 35/744* *(2015.01)*     *A61K 35/747* *(2015.01)*
*C12R 1/25* *(2006.01)*     *A23L 33/135* *(2016.01)*
*A61P 1/14* *(2006.01)*     *A61P 3/00* *(2006.01)*

(86) Numéro de dépôt international:
**PCT/EP2015/060753**

(87) Numéro de publication internationale:
**WO 2015/173386 (19.11.2015 Gazette 2015/46)**

(54) **COMPOSITION DE LACTOBACILLUS PERMETTANT DE FAVORISER LA CROISSANCE JUVENILE HUMAINE ET ANIMALE EN CAS DE MALNUTRITION**

LACTOBACILLUS-ZUSAMMENSETZUNG ZUR STIMULIERUNG DES JUGENDWACHSTUMS VON MENSCHEN UND TIEREN BEI MANGELERNÄHRUNG

LACTOBACILLUS COMPOSITION ALLOWING THE STIMULATION OF HUMAN AND ANIMAL JUVENILE GROWTH IN CASES OF MALNUTRITION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **16.05.2014 FR 1454422**

(43) Date de publication de la demande:
**10.05.2017 Bulletin 2017/19**

(73) Titulaires:
• **Ecole Normale Supérieure de Lyon**
  **69007 Lyon (FR)**
• **Université Claude Bernard Lyon 1**
  **69100 Villeurbanne (FR)**
• **Centre National de la Recherche Scientifique (C.N.R.S.)**
  **75016 Paris (FR)**

(72) Inventeurs:
• **LEULIER, François**
  **F-69360 Serezin du Rhone (FR)**
• **STORELLI, Gilles**
  **F-69003 Lyon (FR)**
• **SCHWARZER, Martin**
  **741 01 Novy Jicin (CZ)**
• **MARTINO, Maria Elena**
  **F-69007 Lyon (FR)**

(74) Mandataire: **Regimbeau**
  **20, rue de Chazelles**
  **75847 Paris Cedex 17 (FR)**

(56) Documents cités:
**WO-A1-2013/057063**     **WO-A1-2013/191845**

• **AGUSTINA RINA ET AL: "Probiotics Lactobacillus reuteri DSM 17938 and Lactobacillus casei CRL 431 Modestly Increase Growth, but Not Iron and Zinc Status, among Indonesian Children Aged 1-6 Years", JOURNAL OF NUTRITION, vol. 143, no. 7, juillet 2013 (2013-07), pages 1184-1193, XP002735351,**
• **OBERHELMAN R A ET AL: "A placebo-controlled trial of Lactobacillus GG to prevent diarrhea in undernourished Peruvian children", JOURNAL OF PEDIATRICS, MOSBY-YEAR BOOK, ST. LOUIS, MO, US, vol. 134, no. 1, 1 janvier 1999 (1999-01-01), pages 15-20, XP027487370, ISSN: 0022-3476, DOI: 10.1016/S0022-3476(99)70366-5 [extrait le 1999-01-01]**
• **WANG J ET AL: "Lactobacillus plantarum ZLP001: In vitro Assessment of Antioxidant Capacity and Effect on Growth Performance and Antioxidant Status in Weaning Piglets", ASIAN-AUSTRALASIAN JOURNAL OF ANIMAL SCIENCES, vol. 25, no. 8, août 2012 (2012-08), pages 1153-1158, XP002735352,**

- SON V M ET AL: "Dietary administration of the probiotic, Lactobacillus plantarum, enhanced the growth, innate immune responses, and disease resistance of the grouper Epinephelus coioides", FISH AND SHELLFISH IMMUNOLOGY, ACADEMIC PRESS, LONDON, GB, vol. 26, no. 5, 1 mai 2009 (2009-05-01), pages 691-698, XP026097888, ISSN: 1050-4648, DOI: 10.1016/J.FSI.2009.02.018 [extrait le 2009-03-03]
- DATABASE EMBASE [Online] ELSEVIER SCIENCE PUBLISHERS, AMSTERDAM, NL; 1994, GONZALEZ S N ET AL: "Biotherapeutic role of fermented milk", XP002735353, Database accession no. EMB-1996012348 & GONZALEZ S N ET AL: "Biotherapeutic role of fermented milk", BIOTHERAPY 1994 NL, vol. 8, no. 2, 1994, pages 129-134, ISSN: 0921-299X
- ERKOSAR BERRA ET AL: "Host-Intestinal Microbiota Mutualism: "Learning on the Fly"", CELL HOST & MICROBE, vol. 13, no. 1, janvier 2013 (2013-01), pages 8-14, XP002735354,
- GILLES STORELLI ET AL: "PromotesSystemic Growth by Modulating Hormonal Signals through TOR-Dependent Nutrient Sensing", CELL METABOLISM, CELL PRESS, UNITED STATES, vol. 14, no. 3, 14 juillet 2011 (2011-07-14), pages 403-414, XP028388621, ISSN: 1550-4131, DOI: 10.1016/J.CMET.2011.07.012 [extrait le 2011-08-10]
- anonyme: "Qu'est-ce que la malnutrition?", OMS , 24 January 2017 (2017-01-24), Retrieved from the Internet: URL:https://www.who.int/features/qa/malnutrition/fr/ [retrieved on 2019-08-16]

Remarques:
Le dossier contient des informations techniques présentées postérieurement au dépôt de la demande et ne figurant pas dans le présent fascicule.

**Description**

La présente invention concerne une composition pharmaceutique ou probiotique permettant de favoriser la croissance juvénile humaine et animale en cas de malnutrition chez les mammifères. Cette composition comprend comme principe actif ou ingrédient au moins une bactérie à tropisme intestinal, de préférence une bactérie lactique.

Décrit comme « un organe additionnel », la communauté microbienne intestinale (ou microbiote intestinal) joue un rôle clé bénéfique pour l'hôte en exerçant de nombreuses fonctions biologiques, telles que l'aide à l'efficacité de la digestion, le métabolisme des substrats, la lutte contre les pathogènes, ou encore la mise en place et l'homéostasie des réponses immunitaires. Les déséquilibres entre différentes populations bactériennes intestinales ont des répercussions parfois délétères, conduisant au développement de diverses pathologies comme des maladies inflammatoires chroniques ou des désordres métaboliques dont l'obésité ou encore le diabète de type 2. Ces déséquilibres sont également potentiellement impliqués dans le développement de cancers et la mise en place de syndromes comportementaux. Le maintien de l'équilibre du microbiote intestinal est donc essentiel : influencer sa composition et/ou son activité serait un atout majeur pour le traitement des syndromes précités. Dans cet esprit, l'idée de l'utilisation de souches bactériennes dites « probiotiques » pour intervenir sur des pathologies influencées par le microbiote trouve tout son sens.

Définis en 2001 par l'Organisation mondiale de la Santé (OMS) et l'Organisation des Nations Unies pour l'alimentation et l'agriculture (FAO), les probiotiques sont des «micro-organismes vivants, qui, lorsqu'ils sont ingérés en quantité suffisante, exercent des effets positifs sur la santé, au-delà des effets nutritionnels traditionnels ».

La présente invention s'inscrit quant à elle dans le rétablissement de conditions permettant d'optimiser la croissance juvénile ou de restaurer une croissance juvénile normale dans un contexte de malnutrition chez les mammifères.

La présente invention a ainsi pour objectif de proposer de nouvelles compositions permettant de favoriser la croissance juvénile ou de rétablir une croissance juvénile normale chez des mammifères, humains ou animaux, ayant été ou étant soumis à une malnutrition, notamment une sous-alimentation et/ou une mauvaise assimilation.

Un autre objectif de l'invention est de fournir de telles compositions, basées sur l'emploi de souches bactériennes ayant un tropisme intestinal, notamment de souches commensales, ou acceptables en tant que probiotique.

Un autre objectif de l'invention est de fournir de telles compositions pouvant aussi s'inscrire dans un contexte thérapeutique.

Un autre objectif encore de l'invention est d'offrir une méthode de traitement probiotique ou thérapeutique.

Un autre objectif encore de l'invention est de fournir de telles compositions et méthodes permettant de supporter une allégation santé et/ou nutritionnelle en accord avec la législation en vigueur, notamment la législation européenne.

L'invention est basée sur le fait que certaines souches de bactéries ayant un tropisme intestinal chez une espèce animale ont un effet favorisant ou rétablissant la croissance juvénile chez un sujet de cette même espèce ou d'une autre espèce qui est soumis à une malnutrition, notamment une sous-alimentation ou une mauvaise assimilation. Il a pu ainsi être démontré que des souches bactériennes du genre *Lactobacillus* étaient capables de favoriser la croissance juvénile aussi bien dans un modèle drosophile que dans un modèle souris couplés à des régimes nutritionnels carencés, et en outre un lien a pu être établi entre ces résultats chez la souris et une augmentation du titre sérique d'IGF-1 chez les souris traitées dans ces conditions avec ces bactéries.

La présente invention a donc pour objet une composition comprenant au moins une souche de *Lactobacillus* ayant un tropisme intestinal choisie parmi *L. plantarum* et *L. fermentum,* pour utilisation pour favoriser la croissance juvénile dans le traitement de la malnutrition des mammifères. La notion de malnutrition regroupe la sous-alimentation, la suralimentation et la mauvaise assimilation. L'invention vise en particulier la sous-alimentation et/ou la mauvaise assimilation. Elle vise de préférence la sous-alimentation. L'indication de la composition pourra être thérapeutique ou santé, comme médicament, ou nutritionnelle, comme probiotique.

Par bactérie ayant un « tropisme intestinal », on entend une bactérie à la capacité de passer la barrière gastrique et qui est capable de persister dans l'intestin.

Conformément à une caractéristique avantageuse de l'invention, la bactérie peut favoriser la production d'IGF-1 chez l'homme ou l'animal qui est traité par la composition conforme à l'invention.

Sont notamment décrites des souches de bactérie appartenant aux familles suivantes : Lactobacillaceae, Streptoccaceae, Enterococcaceae, Leuconostocaceae, Bifidobacteriaceae, parmi lesquelles une ou des souches du genre *Lactobacillus,* en particulier de l'une des espèces suivantes, *Lactobacillus delbrueckii, Lactobacillus plantarum, Lactobacillus fermentum, Lactobacillus casei, Lactobacillus paracasei, Lactobacillus rhamnosus.*

Plus particulièrement, sont décrites des bactéries appartenant aux espèces *Lactobacillus plantarum, Lactobacillus fermentum, Lactobacillus casei, Lactobacillus paracasei, Lactobacillus rhamnosus,* et notamment *L. casei* ATCC 393, *L. casei* L919, *L. paracasei* ATCC25302, *L. paracasei* Shirota *L. rhamnosus* L900, *L. rhamnosus* L908, *L. rhamnosus* GG. Selon l'invention, la souche est choisie parmi les espèces *Lactobacillus plantarum et Lactobacillus fermentum,* Suivant un mode de réalisation de l'invention, la souche de bactérie est choisie parmi *L. plantarum* WJL, *L. plantarum* G821, *L. fermentum* ATCC9338,.

Dans un mode de réalisation spécifique, il s'agit de bactéries de l'espèce *Lactobacillus plantarum,* par exemple la souche WJL ou la souche G821, déposée à la Collection Nationale de Culture de Microorganismes (Institut Pasteur) sous le numéro d'enregistrement CNCM I-4979 le 11 mai 2015. La souche G821 a été obtenue par évolution expérimentale (c.a.d. par accumulation et sélection de variants naturels) de la souche *L. plantarum* NIZO2877.

Les compositions selon l'invention comprennent au moins une souche de bactérie choisie parmi les espèces *Lactobacillus plantarum* et *Lactobacillus fermentum,* qui présente les propriétés requises et permet de favoriser ou rétablir une croissance juvénile en dépit de l'épisode de malnutrition. Bien entendu, la composition selon l'invention peut comprendre plus d'une souche de bactérie répondant aux besoins de l'invention. Notamment, la composition comprend 2 ou plus de ces souches de bactéries, choisies dans une même espèce ou dans des espèces différentes.

Suivant un mode de réalisation intéressant, la bactérie est un *L. plantarum.* Des souches appropriées sont *L. plantarum* G821 et *L. plantarum* WJL (Eun-Kyoung Kim et al., Genome Announcements, November/December 2013, vol. 1, n° 6 e00937-13, GenBank AUTE00000000, *Lactobacillus plantarum* WJL, whole genome shotgun sequencing project). Cette souche WJL a été initialement isolée et peut être isolée de la drosophile (JH Ryu et al., Science 2008, 319 : 777-782).

D'autres exemples de souches qui sont également décrites sont les suivantes : *L. casei* ATCC 393, *L. casei* L919 (Koryszewska-Baginska A. et al., 26 septembre 2013, Genome Announc), *L. paracasei* ATCC25302, *L. paracasei* Shirota (Yuki N et al., Int J Food Microbiol. 1er avril 1999;48(1) :51-7), *L. fermentum* ATCC9338, *L. rhamnosus* L900 (Aleksandrzak-Piekarczyk T. et al., Genome Announc, 15 août 2013), *L. rhamnosus* L908 (Koryszewska-Baginska A. et al., 20 février 2014, Genome Announc), *L. rhamnosus* GG (Kankainen M. et al., Proc Natl Acad Sci USA, 6 octobre 2009).

La présente invention apporte donc à la technique l'enseignement que des souches bactériennes à tropisme intestinal favorisent la croissance juvénile notamment chez des mammifères (humains ou animaux) malnutris. Mais l'invention ne se limite pas à cet enseignement, elle donne en outre à l'homme du métier de déterminer de manière sûre les souches de bactéries utiles pour l'invention. Différents critères peuvent être à la base de tests, en étant utilisés seuls ou en association. Parmi ces critères, on notera le taux sérique d'IGF-1 chez l'animal modèle (e.g. souris), la croissance larvaire chez *Drosophila melanogaster,* la croissance de souris modèles illustrée, par exemple, par la taille des fémurs ou la prise de taille des animaux. Sur la base de ces critères ou de critères similaires, il est possible pour l'homme du métier de mettre au point des tests comparant des individus élevés en présence ou en l'absence de la bactérie à tester, dans des conditions de malnutrition. L'invention apporte un plus en proposant des modèles animaux axéniques et l'élevage des animaux en présence ou en l'absence de la bactérie ou des bactéries à tester permettant de concentrer le test sur l'analyse des propriétés intrinsèques de la ou les souches testées et ainsi de s'affranchir de l'effet que le microbiote intestinal résidant d'animaux conventionnels pourrait avoir dans le contexte du test fonctionnel. Il en résulte une prédictivité accrue du test quant au potentiel fonctionnel de la souche testée.

On entend par organisme « axénique » un organisme (e.g. drosophile, souris) élevé dans un environnement dépourvu de microorganismes et donc dépourvu de flore intestinale.

On entend par organisme « monoxénique » un organisme (e.g. drosophile, souris) axénique associé élevé en présence d'un unique microorganisme et donc porteur de cet unique microorganisme en guise de flore intestinale.

Dans le cadre de l'invention, les souches de bactérie présentes dans les compositions selon l'invention se caractérisent par le fait qu'elles répondent positivement à, ou ont été sélectionnées en utilisant, un test dans lequel on compare la croissance de larves issues de drosophiles axéniques sur un milieu nutritif carencé en levure (et donc en protéines) par rapport à un milieu nutritif conventionnel. Les milieux nutritionnels conventionnels de laboratoire comprennent généralement a *minima* une farine végétale, typiquement de maïs, de la levure inactivée, typiquement de la levure de bière ou de la levure de boulanger (*Saccharomyces cerevisiae*), de l'agar et de l'eau. Un milieu carencé en levure inactivée ne permet pas un développement post-embryonnaire (c.a.d. juvénile) optimum des larves de drosophiles, comme il a été rapporté dans G. Storelli et al., Cell Metabolism 14, 403-414, 2011. Un groupe d'embryons est en outre ensemencé avec la bactérie à tester, ce qui permet aux larves émergeant des embryons d'être associées à la bactérie à tester. On peut donc détecter si cette bactérie est susceptible de favoriser ou permettre la croissance juvénile malgré un milieu nutritif carencé en levure.

Le test drosophile est typiquement mené de la manière suivante (Test A) :

- on dispose d'un lot d'embryons issus de parents *Drosophila melanogaster* (e.g. souche *Drosophila yw,* ou toute autre souche dites "sauvage") axéniques ;
- à J1 : on répartit ces embryons en au moins 2 groupes (40 embryons, réalisé en triplicat pour un total de 120 embryons par groupe) sur un milieu nutritionnel comprenant de la farine de maïs de l'agar et de l'eau, carencé en levure, l'un des groupes d'embryons est en outre inoculé par une suspension d'environ $10^8$ CFU de la bactérie à tester dans un tampon salin (e.g. PBS), ce qui forme le groupe monoxénique, l'autre groupe formant le groupe axénique, on conduit l'élevage à environ 25°C jusqu'à J7 ; typiquement le milieu de culture est constitué, pour 1 litre de milieu, de 7,14 g d'agar, 80 g de farine de maïs et 6 g de levure inactivée, le milieu est cuit dans l'eau bouillante pendant 10 min, puis refroidissement ; typiquement, le même milieu, non carencé en levure inactivée, contient 50 g de levure inactivée ;

- à J7 : on récupère au minimum 60 larves de drosophiles de chaque groupe issues des embryons inoculés ou non (respectivement larves monoxéniques et larves axéniques), on leur applique un choc thermique rapide (e.g. 5 secondes ; typiquement les larves sont placées 5 secondes sur une plaque chauffée à 100°C; il s'agit de tuer les larves sans les déformer pour permettre la mesure de leur taille) ;

- on détermine la moyenne de la longueur des larves de chacun des groupes, et on compare les moyennes obtenues ;

- la souche de bactérie testée est considérée comme répondant positivement au test si la moyenne de la longueur des larves du groupe monoxénique est supérieure à la moyenne de la longueur des larves du groupe axénique avec une valeur de p inférieure ou égale à 0,001, de préférence inférieure ou égale à 0,0001 dans le test statistique de Mann-Whitney, réalisé sur l'ensemble du jeu de données des tailles des larves des deux groupes.

La présente invention a donc pour objet une composition comprenant au moins une souche de *Lactobacillus* ayant un tropisme intestinal choisie parmi les espèces *Lactobacillus plantarum* et *Lactobacillus fermentum,* pour utilisation pour favoriser la croissance juvénile dans le traitement de la malnutrition des mammifères, dans laquelle la souche de bactérie répond positivement au test sur drosophile tel que décrit ci-dessus (Test A).

A titre d'exemples, on peut citer les souches suivantes : *L. plantarum* WJL, *L. plantarum* G821, *L. fermentum* ATCC9338,. D'autres souches peuvent être identifiées parmi les bactéries à tropisme intestinal et notamment parmi les espèces mentionnées plus haut, et en particulier *L. casei* ATCC 393, *L. casei* L919, *L. paracasei* ATCC25302, *L. paracasei* Shirota, *L. rhamnosus* L900, *L. rhamnosus* L908, *L. rhamnosus* GG..

Dans un mode de réalisation de l'invention, la souche WJL ou toute autre souche ayant un effet « marqué » (par exemple *L. casei* ATCC 393, *L. fermentum* ATCC 9338, *L. paracasei* ATCC 25302) est utilisée comme souche de référence afin d'identifier et sélectionner des souches bactériennes ayant un effet « marqué » sur la croissance juvénile, à savoir un effet proche de celui de cette souche de référence, e.g. WJL (effet non significativement différent de la souche de référence, e.g. WJL), ou un effet « fort » sur la croissance juvénile (effet significativement supérieur à la souche de référence, e.g. WJL).

Pour ce faire, le test drosophile (incluant 60 larves par condition) est appliqué à la souche de référence, e.g. WJL et à la souche à tester (en parallèle, de préférence, ou alors on peut disposer de données de référence préalablement générées pour la souche de référence, e.g. WJL, par exemple les données présentées dans les exemples). On compare ensuite les moyennes obtenues pour les deux souches. La souche de bactérie testée est considérée comme étant une souche à effet marqué si la moyenne de la longueur des larves du groupe monoxénique n'est pas significativement différente de la moyenne de la longueur des larves du groupe de référence, e.g. WJL avec une valeur de p supérieure à 0,001 dans le test statistique de Mann-Whitney, réalisé sur l'ensemble du jeu de données. L'effet est fort si ladite moyenne pour la souche à tester est significativement supérieure à la moyenne pour la souche de référence, e.g. WJL, ce qui est le cas lorsque la valeur de p du test statistique est inférieure ou égale à 0,001, de préférence à 0,0001. L'effet est qualifié d'intermédiaire si ladite moyenne pour la souche à tester est significativement inférieure à la moyenne pour la souche de référence, e.g. WJL, ce qui est le cas lorsque la valeur de p du test statistique est inférieure ou égale à 0,001, de préférence à 0,0001.

La présente invention a donc pour objet une composition comprenant au moins une souche de *Lactobacillus* ayant un tropisme intestinal choisie parmi les espèces *Lactobacillus plantarum* et *Lactobacillus fermentum,* pour utilisation pour favoriser la croissance juvénile dans le traitement de la malnutrition des mammifères, dans laquelle la souche de bactérie a un effet marqué ou fort sur la croissance dans le modèle drosophile tel que décrit ci-dessus.

A titre d'exemples, on peut citer les souches suivantes : *L. plantarum* WJL, *L. plantarum* G821, *L. fermentum* ATCC9338,. D'autres souches peuvent être identifiées parmi les bactéries à tropisme intestinal et notamment parmi les espèces mentionnées plus haut, et en particulier *L. casei* ATCC 393, *L. casei* L919, *L. paracasei* ATCC25302, *L. paracasei* Shirota, *L. rhamnosus* L900, *L. rhamnosus* L908, *L. rhamnosus* GG..

L'invention a également pour objet une composition comprenant une souche de bactérie à tropisme intestinal du genre *Lactobacillus,* choisie parmi les espèces *L. Plantarum* et *L. fermentum,* pour une utilisation visant à favoriser la croissance juvénile dans le traitement de la malnutrition des mammifères, dans laquelle la souche de bactérie répond positivement au test suivant (Test D):

- on dispose d'un lot d'embryons de parents *Drosophila melanogaster* axéniques ;

- à J1 : on répartit ces embryons en au moins 2 groupes (40 embryons, réalisé en triplicat soit 120 embryons par groupe) sur un milieu nutritionnel comprenant de la farine de maïs, de l'agar et de l'eau, carencé en levure, l'un des groupes d'embryons est inoculé par une suspension d'environ $10^8$ CFU de ladite bactérie dans un tampon salin, ce qui forme le groupe monoxénique test, l'autre groupe étant inoculé par une suspension d'environ $10^8$ CFU de bactérie de référence *L. plantarum* WJL, *L. fermentum* ATCC 9338, dans un tampon salin, formant le groupe monoxénique de référence, on conduit l'élevage des deux groupes à environ 25°C jusqu'à J7 ;

- à J7 : on récupère au minimum 60 larves de drosophiles de chaque groupe, on leur applique un choc thermique;
- on détermine la moyenne de la longueur des larves de chacun des groupes, et on compare les moyennes obtenues ;
- ladite souche de bactérie ayant, par rapport à la bactérie de référence, un effet fort avec une moyenne de la longueur des larves du groupe monoxénique test avec cette souche de bactérie significativement supérieure à la moyenne pour le groupe monoxénique avec la souche de référence, avec une valeur de p est inférieure ou égale à 0,001, de préférence à 0,0001 dans le test statistique de Mann-Whitney, réalisé sur l'ensemble du jeu de données des tailles des larves des deux groupes.

Selon l'invention, il est aussi possible de déterminer si une souche de bactérie à tropisme intestinal permet de favoriser la croissance en cas de malnutrition, en utilisant un modèle souris axénique qui permet de réaliser un suivi de croissance squelettique des souris en présence de la bactérie à tester en comparaison avec l'absence de microbiote et/ou avec la présence d'une souche de bactérie de référence. Ce modèle peut être utilisé en première intention ou sur des souches ayant répondu positivement au test drosophile.

Suivant une caractéristique de l'invention, les souches de bactérie présentes dans les compositions selon l'invention se caractérisent par le fait qu'elle répond positivement au test de croissance squelettique suivant (Test B) :

- à partir d'une même lignée de souris (typiquement souris Balb/c) on établit une lignée de souris parentales axéniques et une lignée de souris parentales monoxéniques (associées à la bactérie à tester), et l'on produit des juvéniles qui sont élevées avec les parents en milieu nutritionnel conventionnel jusqu'à leur sevrage (à J21) ; pour former le groupe des juvéniles monoxéniques, on utilise des parents mono-associés avec la souche de bactérie à tester,
- à J21 : on dispose de 8 juvéniles sevrées issues de chacune de ces deux lignées, formant le groupe monoxénique et le groupe axénique, et on les élève en régime nutritionnel carencé en protéines (8%) et en lipides (2,5%); typiquement, le régime nutritionnel conventionnel comprend 23% de protéines et 5% de lipides,
- à J 56 : on détermine la moyenne des tailles des souris pour un groupe considéré par la mesure de l'extrémité du museau à la base de la queue de chaque individu ; une autre mesure possible consiste à sacrifier les individus, à en prélever les fémurs et à mesurer ces derniers,
- la souche de bactérie lactique étant considérée comme répondant positivement au test si la taille moyenne des individus et/ou des fémurs, du groupe monoxénique, est supérieur à la taille moyenne, respectivement, des individus et/ou des fémurs, du groupe axénique, avec une valeur de p inférieure ou égale à 0,05, dans le test statistique de Tukey.

La présente invention a donc pour objet une composition comprenant au moins une souche de Lactobacillus ayant un tropisme intestinal, choisie parmi les espèces *L. plantarum* et *L. fermentum,* pour utilisation pour favoriser la croissance juvénile dans le traitement de la malnutrition des mammifères, dans laquelle la souche de bactérie répond positivement au test de croissance squelettique sur souris (Test B). Le test drosophile permet un criblage plus rapide, de sorte que généralement, l'on dispose aussi du test sur drosophile, et la souche de bactérie répond positivement aux tests drosophile et de croissance squelettique.

A titre d'exemple, on peut citer la souche suivante : *L. plantarum* WJL. On peut également citer les souches *L. plantarum* G821 et *L. fermentum* ATCC9338. D'autres souches peuvent être identifiées parmi les bactéries à tropisme intestinal et notamment parmi les espèces et souches mentionnées plus haut, et en particulier parmi les souches *L. casei* ATCC 393, *L. casei* L919, *L. paracasei* ATCC25302, *L. paracasei* Shirota, *L. rhamnosus* L900, *L. rhamnosus* L908, *L. rhamnosus* GG.

Dans un mode de réalisation de l'invention, la souche WJL ou une autre souche à effet « marqué » est utilisée comme souche de référence afin d'identifier et de sélectionner des souches bactériennes ayant un effet « marqué » sur la croissance juvénile, à savoir un effet proche de celui de cette souche de référence, e.g. WJL (effet non significativement différent de la souche de référence, e.g. WJL), ou un effet « fort » sur la croissance juvénile (effet significativement supérieur à la souche de référence, e.g. WJL).

Pour ce faire, le test souris (incluant 8 souris par condition) est appliqué à la souche de référence, e.g. WJL et à la souche à tester (en parallèle, de préférence, ou alors on peut disposer de données de référence préalablement générées pour la souche WJL, par exemple les données présentées dans les exemples). On compare ensuite les moyennes obtenues pour les deux souches. La souche de bactérie testée est considéré comme étant une souche à effet marqué si la moyenne de la taille moyenne des individus et/ou des fémurs du groupe monoxénique n'est pas significativement différente de la moyenne correspondante pour le groupe de référence, e.g. WJL avec une valeur de p supérieure à 0,05 dans le test statistique de Tukey. L'effet est fort si ladite moyenne pour la souche à tester est significativement supérieure à la moyenne pour la souche de référence, e.g. WJL, ce qui est le cas lorsque la valeur de p du test statistique est inférieure ou égale à 0,05. L'effet est qualifié d'intermédiaire si ladite moyenne pour la souche à tester (qui a été qualifiée par rapport aux souris axéniques dans le test précédent) est significativement inférieure à la moyenne pour la souche de référence, e.g. WJL, ce qui est le cas lorsque la valeur de p du test statistique est inférieure ou égale à 0,05.

La composition selon l'invention comprendra de préférence au moins une souche de bactérie choisie parmi les espèces *L. plantarum* et *L. fermentum* ayant un tel effet marqué ou fort.

Les souches de bactérie selon l'invention peuvent se caractériser par le fait qu'elles ont un impact positif sur le taux d'IGF-1 sérique. C'est ainsi qu'il a été possible, sur la base d'un modèle de souris axéniques, de montrer que des souris élevées en milieu à faible teneur en protéines mais en présence de la bactérie (souris monoxéniques) avaient une croissance supérieure et en même temps un taux d'IGF-1 sérique supérieur, par rapport à ces mêmes souris élevées avec ce milieu carencé en protéines et en l'absence de la bactérie (souris axéniques). Ceci permet de proposer un test permettant de déterminer si une souche de bactérie a le potentiel à augmenter le taux sérique d'IGF-1, ce test pouvant être appliqué en association avec un test de croissance squelettique afin de préciser ou d'affiner l'effet de la souche dans ce contexte de croissance juvénile, ou en première intention.

Dans ce cas, les souches de bactérie présentes dans les compositions selon l'invention se caractérisent par le fait qu'elles répondent positivement au test du taux d'IGF-1 sérique suivant (Test C) :

- à partir d'une même lignée de souris (typiquement souris Balb/c) on établit une lignée de souris parentales axéniques et une lignée de souris parentales monoxéniques (associées à la bactérie à tester), et l'on produit des juvéniles qui sont élevées avec les parents en milieu nutritionnel conventionnel jusqu'à leur sevrage (à J21) ; pour former le groupe des juvéniles monoxéniques, on utilise des parents mono-associés avec la souche de bactérie à tester,
- à J21 : on dispose de 8 juvéniles sevrées issues de chacune de ces deux lignées, formant le groupe monoxénique et le groupe axénique, et on les élève en régime nutritionnel carencé en protéines (8%) et en lipides (2,5%); typiquement, le régime nutritionnel conventionnel comprend 23% de protéines et 5% de lipides,
- à J 56 : on prélève du sang des juvéniles de chaque groupe et on détermine le taux sérique moyen d'IGF-1 pour chaque groupe; cette mesure du taux sérique d'IGF-1 est de préférence réalisée sur du sérum dilué (1:25) ; on utilise de préférence des kits ELISA commerciaux de détection de l'IGF-1 en suivant les instructions du fabricant,
- la souche de bactérie lactique est considérée comme répondant positivement au test si le taux sérique moyen d'IGF-1 du groupe monoxénique est supérieur au taux sérique moyen du groupe axénique avec une valeur de p inférieure ou égale à 0,05 dans le test statistique de Tukey.

La présente invention a donc pour objet une composition comprenant au moins une souche de *Lactobacillus* ayant un tropisme intestinal choisie parmi les espèces *L. plantarum* et *L. fermentum,* pour utilisation pour favoriser la croissance juvénile dans le traitement de la malnutrition des mammifères, dans laquelle la souche de bactérie augmente le taux d'IGF-1 sérique (Test C). Il s'agit notamment d'une souche de bactérie qui répond positivement au test sur souris tel que décrit ci-dessus.

La présente invention a aussi pour objet une composition comprenant au moins une souche de *Lactobacillus* ayant un tropisme intestinal choisie parmi les espèces *L. plantarum* et *L. fermentum,* pour utilisation pour favoriser la croissance juvénile dans le traitement de la malnutrition des mammifères, dans laquelle la souche de bactérie répond positivement au test de croissance squelettique sur souris et augmente le taux sérique d'IGF-1 chez ces mêmes souris, notamment ladite souche répond positivement également au test sur drosophile.

A titre d'exemple, on peut citer la souche suivante : *L. plantarum* WJL. On peut également citer les souches *L. plantarum* G821 ou *L. fermentum* ATCC9338. D'autres souches peuvent être identifiées parmi les bactéries à tropisme intestinal et notamment parmi les espèces et souches mentionnées plus haut, notamment parmi les souches *L. casei* ATCC 393, *L. casei* L919, *L. paracasei* ATCC25302, *L. paracasei* Shirota, *L. rhamnosus* L900, *L. rhamnosus* L908, *L. rhamnosus* GG.

Dans un mode de réalisation de l'invention, la souche WJL ou une autre souche à effet « marqué» est utilisée comme souche de référence afin d'identifier et sélectionner des souches bactériennes ayant un effet « marqué » sur le taux sérique d'IGF-1, à savoir un effet proche de celui de cette souche de référence, e.g. WJL (effet non significativement différent de la souche de référence, e.g. WJL), ou un effet « fort » sur le taux sérique d'IGF-1 (effet significativement supérieur à la souche de référence, e.g. WJL).

Pour ce faire, le test souris (incluant 8 souris par condition) de mesure du taux sérique d'IGF-1 est appliqué à la souche de référence, e.g. WJL et à la souche à tester (en parallèle, de préférence, ou alors on peut disposer de données de référence préalablement générées pour la souche de référence, e.g. WJL, par exemple les données présentées dans les exemples). On compare ensuite les moyennes de taux sérique d'IGF-1 obtenues pour les deux souches. La souche de bactérie testée est considérée comme étant une souche à effet marqué si la moyenne des taux d'IGF-1 du groupe monoxénique n'est pas significativement différente de la moyenne pour le groupe de référence, e.g. WJL avec une valeur de p supérieure à 0,05, dans le test statistique de Tukey. L'effet est fort si ladite moyenne pour la souche à tester est significativement supérieure à la moyenne pour la souche de référence, e.g. WJL lorsque la valeur de p est inférieure ou égale à 0,05. L'effet est qualifié d'intermédiaire si ladite moyenne pour la souche à tester (préalablement qualifiée sur le test axénique précédent) est significativement inférieure à la moyenne pour la souche de référence, e.g.

WJL lorsque la valeur de p est inférieure ou égale à 0,05. La composition selon l'invention comprendra donc de préférence une souche de bactérie ayant un tel effet marqué ou fort, et en particulier cette souche de bactérie a un effet marqué ou fort à la fois sur la croissance squelettique et sur le taux sérique d'IGF-1.

La composition selon l'invention, comprenant au moins une souche de bactérie conforme à l'invention, est apte à une utilisation comme médicament destiné à favoriser la croissance juvénile dans le traitement de la malnutrition des mammifères caractérisée par une carence protéique. Suivant une caractéristique de l'invention, le sujet à traiter, malnutri ou ayant été malnutri, peut avoir un taux sérique d'IGF-1 inférieur à celui rencontré chez des individus de même âge et sexe et non mal nourris.

La composition est utilisable chez l'homme de la naissance à la puberté.

La composition est utilisable chez les mammifères, notamment animaux de rente (bovins, ovins, caprins, porcins, aviaire), les animaux de compagnie (chien, chat) et de sport (cheval, dromadaire, chameau), entre le sevrage et la maturité sexuelle.

La composition contient une quantité de $10^5$ à $10^{12}$, notamment de $10^6$ à $10^{12}$, de préférence de $10^8$ à $10^{12}$ cellules bactériennes formant des colonies (CFU) selon l'invention, par gramme de composition. Le terme CFU signifie « colony forming units » selon l'expression technique anglaise. Par gramme de composition, on entend de préférence la composition pharmaceutique ou la composition probiotique formée des bactéries, co-ingrédients, et excipients ou vecteurs. Par cellules bactériennes, on entend une souche de bactérie unique conforme à l'invention ou un mélange d'au moins deux bactéries, conformes à l'invention.

La composition selon l'invention comprend la ou les bactéries lactiques sous forme vivante. Il peut s'agir d'une suspension bactérienne, qui peut être congelée et décongelée avant usage, ou encore une poudre lyophilisée, pouvant être utilisée telle quelle ou après reprise dans un véhicule approprié. Elle peut alors comprendre un excipient de lyophilisation classique.

La composition peut être une forme d'administration orale (par exemple poudre, gélule, comprimé) éventuellement sous une forme gastro-protégée permettant de passer l'estomac et libérer les bactéries dans l'intestin.

Les compositions selon l'invention sont donc utiles pour le traitement thérapeutique pour favoriser la croissance juvénile en cas de malnutrition des mammifères, comprenant l'administration à un patient, humain (de la naissance à la puberté) ou animal (du sevrage à la maturité sexuelle), qui en a besoin, c'est-à-dire malnutri ou ayant subi une période de malnutrition, notamment avec carence protéique. Le patient qui en a besoin peut avoir un taux sérique d'IGF-1 inférieur à celui rencontré chez des individus de même âge et sexe et non mal nourris.

Comme il est indiqué, le traitement thérapeutique comprend l'administration d'une quantité suffisante d'une composition selon l'invention telle que décrite ci-dessus. La quantité et la fréquence d'administration dépendront notamment de la sévérité de la malnutrition, de l'âge et de l'état du patient. La méthode comprendra l'administration en une ou plusieurs fois, pouvant être échelonnée sur la période de croissance du sujet (jusqu'à la puberté ou la maturité sexuelle), de doses de composition selon l'invention. Les doses peuvent être fractionnées pour faciliter l'administration, notamment en fonction de l'âge du patient. La fréquence d'administration est notamment comprise entre une dose (unique ou fractionnée) tous les jours et une dose tous les mois. Typiquement, la fréquence d'administration sera comprise entre une dose (unique ou fractionnée) tous les jours et une dose toutes les semaines, voire tous les 2, 3, 4, 5 ou 6 jours. Chaque dose (unique ou fractionnée) représente notamment plusieurs grammes à plusieurs dizaines de gramme de composition. La méthode comprend notamment l'administration d'une composition comprenant une quantité de $10^5$ à $10^{12}$, notamment de $10^6$ à $10^{12}$, de préférence de $10^8$ à $10^{12}$ cellules bactériennes formant des colonies CFU par gramme de composition (par cellules bactériennes, on entend une souche de bactérie unique conforme à l'invention ou un mélange d'au moins deux bactéries, conformes à l'invention). La méthode comprend notamment l'administration d'une composition comprenant la ou les bactéries lactiques sous forme vivante. Cette composition peut être une suspension bactérienne, qui peut être congelée et décongelée avant usage, ou encore une poudre lyophilisée, pouvant être utilisée telle quelle ou après reprise dans un véhicule approprié. Cette composition peut alors comprendre un excipient de lyophilisation classique, être une forme d'administration orale (par exemple poudre, gélule, comprimé) éventuellement sous une forme gastro-protégée permettant de passer l'estomac et libérer les bactéries dans l'intestin, ou encore une forme rectale (par exemple suppositoire).

Dans un mode de réalisation, la composition utilisée selon l'invention dans le traitement de la malnutrition des mammifères, comprend au moins une souche de bactérie qui n'est pas naturellement présente (« not-naturally occuring ») chez l'espèce à laquelle le patient à traiter appartient. Ainsi, il peut s'agir d'une bactérie qui n'est pas naturellement présente chez l'homme ou chez l'animal. Dans cette configuration, lorsqu'il y a plusieurs bactéries différentes, il suffit que l'une d'elles ne soit pas naturellement présente. En revanche, cette bactérie s'avère avoir un tropisme intestinal chez le patient à traiter ou dans l'espèce correspondante (homme ou animal selon l'invention) et répond à la définition des souches actives selon l'invention.

L'invention concerne aussi une méthode de criblage de bactéries capables de favoriser la croissance juvénile en cas de malnutrition des mammifères, utilisant un modèle de drosophile axénique et/ou un modèle de souris axénique.

La méthode avec le modèle drosophile comprend les étapes suivantes :

- on dispose d'un lot d'embryons issus de *Drosophila melanogaster* (e.g. souche *Drosophila yw* ou tout autre souche dite "sauvage") axéniques ;
- on répartit ces embryons en au moins 2 groupes sur un milieu nutritionnel comprenant un milieu nutritif pour drosophiles carencé en levure, l'un des groupes d'embryons est en outre inoculé par une suspension de la bactérie à tester, ce qui forme le groupe des individus monoxéniques ; l'autre groupe forme le groupe axénique,
- on conduit l'élevage à température appropriée (généralement environ 25 °C),
- au terme d'une période d'élevage appropriée (généralement environ 7j), on récupère, de préférence en nombre égal, les larves de drosophiles de chaque groupe issues des embryons initialement inoculés ou non (larves axéniques et larves monoxéniques),
- on détermine la moyenne de la longueur des larves de chacun des groupes, et on compare les moyennes obtenues ;
- la souche de bactérie testée est considérée comme répondant positivement au test si la moyenne de la longueur des larves du groupe monoxénique est supérieure à la moyenne de la longueur des larves du groupe axénique avec une valeur de p inférieure ou égale à 0,001, de préférence inférieure ou égale à 0,0001 dans le test statistique de Mann-Whitney, réalisé sur l'ensemble du jeu de données des tailles des larves des deux groupes.

De préférence, la méthode de criblage reprend les caractéristiques du test drosophile qui a été décrit plus haut.

La méthode avec le modèle souris comprend les étapes suivantes :

- on dispose de juvéniles issues de deux lignées issues d'une même souche de souris (typiquement souris Balb/c), à savoir une lignée de souris parentales axéniques et une lignée de souris parentales monoxéniques (associées à la bactérie à tester),
- on les élève en régime nutritionnel carencé en protéines et de préférence aussi en lipides,
- au terme d'une période d'élevage appropriée, on détermine la moyenne des valeurs d'un ou plusieurs paramètres de chaque groupe, ces paramètres étant liés à la croissance (par exemple prise de poids, croissance squelettique par exemple par mesure de la taille des individus ou de la taille de leurs fémurs), et/ou au taux d'IGF-1 sérique,
- la souche de bactérie lactique est considérée comme répondant positivement au test si la valeur moyenne du paramètre mesuré du groupe monoxénique est supérieure à la valeur moyenne du paramètre mesuré du groupe axénique avec une valeur de p inférieure ou égale à 0,05 dans le test statistique de Tukey.

De préférence, la méthode de criblage reprend les caractéristiques du test souris croissance squelettique ou taux sérique d'IGF-1 décrit ci-dessus.

La méthode de criblage peut aussi être un test comparatif avec une souche de référence, par exemple la souche WJL, et ce test reprend alors les caractéristiques décrites ci-dessus pour les tests drosophile ou souris.

La présente invention a aussi pour objet une composition, une culture pure ou un isolat comprenant la souche G821 (CNCM I-4979), sous forme vivante ou tuée, une composition comprenant cette souche vivante dans un milieu de conservation ou de culture, une composition lyophilisée comprenant cette souche et éventuellement un excipient de lyophilisation et/ou un conservateur, une composition comprenant cette souche sous forme tuée dans un milieu de conservation, ou tuée dans un milieu de conservation, une composition administrable à l'homme ou à l'animal, comprenant cette souche vivante ou tuée dans un milieu ou excipient acceptable pour une administration orale ou rectale. Les milieux de conservation, de culture, les excipients de lyophilisation, les conservateurs, les milieux ou excipients acceptables pour une administration orale ou rectale, sont ceux décrits ci-dessus et ceux bien connus de l'homme du métier.

L'invention va être maintenant décrite plus en détail à l'aide de modes de réalisation de l'invention pris à titre d'exemples non limitatifs et se référant au dessin dans lequel :

- la figure 1 est un graphique montrant le taux de croissance (en cm/jour) de souris de J28 à J56 ; GF : souris axéniques ; WJL : souris élevées en présence de la souche *L. plantarum WJL* ; NIZO 2877 : souris élevées en présence de la souche *L. plantarum WJL* NIZO 2877 ; * p<0,05 ; ***p<0,001 ;
- la figure 2 est un graphe montrant la taille des fémurs à J56, chez des souris GF, WJL ou NIZO 2877 ; * p<0,05 ; ***p<0,001 ;
- la figure 3 est un graphe montrant les taux sériques (en ng/ml) de souris males à J56, groupes GF, WJL ou NIZO 2877* p<0,05 ; ***p<0,001 ;
- la figure 4 est un graphique montrant la taille des larves de Drosophile à 7 jours en conditions de sous-nutrition : un groupe contrôle négatif (pas d'ajout de bactérie sur le milieu de croissance après la ponte), et des groupes caractérisés par l'ajout sur le milieu de croissance, de la souche *L. plantarum* NIZO 2877, *WJL* ou G821.

**Exemple 1 : Criblage fonctionnel de bactéries sur *Drosophile* monoxénique :**

J-1 : on emploie des Drosophiles adultes (*D. melanogaster yw*) axéniques ; elles sont placées dans des cages de ponte comportant un fond (type boîte de Pétri) sur lequel est disposé un milieu nutritionnel conventionnel ; la ponte est assurée par le maintien de la population d'adultes dans la cage 1 nuit à 25°C.

J0 : on découpe 6 pièces du milieu nutritionnel de manière à recueillir 6 fois 40 embryons, on place chacune des pièces sur un milieu nutritionnel carencé contenu dans une boîte de Pétri, on inocule trois boîtes avec du PBS 1x stérile puis les trois autres avec une suspension de la bactérie dans du PBS 1x

On incube 7 jours à 25°C.

J7 : On récupère ensuite, à partir de chaque boîte, les larves qui s'y sont développées (l'expérience est exploitable à partir de 20 larves par boîte, nous avons donc ci-après utilisé des groupes de plus de 60 individus). Les larves subissent un traitement thermique choc (5 secondes sur une plaque chauffée à 100°C), on mesure ensuite la taille des larves.

On a appliqué ce protocole à différentes souches de bactéries, et les expériences et résultats sont résumés dans le tableau 1 suivant :

Tableau 1.

| | Tailles des larves à J7 (mm) | | Analyse statistique | | | | Interprétation du test qualitatif (niveau quantitatif) |
|---|---|---|---|---|---|---|---|
| | Moyenne (n>60, mm) | Ecart-type | Comparaison/animaux axéniques | | Comparaison/L. *plantarum WJL* | | |
| | | | Valeur de p (1) | Signification statistique | Valeur de p (1) | Signification statistique | |
| axénique | 2,693 | 0,497 | | | <0,0001 | **** | |
| *Lactobacillus plantarum WJL* | 3,644 | 0,883 | <0,0001 | **** | | | SPM (marqué) |
| *Lactobacillus plantarum NIZO2877* | 3,398 | 0,750 | <0,0001 | **** | 0,0001 | *** | SPM (intermédiaire) |
| *Lactobacillus casei ATCC 393* | 4,391 | 0,802 | <0,0001 | **** | 0,0721 | ns | SPM (marqué) |
| *Lactobacillus fermentum ATCC 9338* | 4,133 | 0,824 | <0,0001 | **** | >0,9999 | ns | SPM (marqué) |
| *Lactobacillus fermentum KLD* | 2,741 | 0,626 | >0,9999 | ns | <0,0001 | **** | Pas d'effet sur la croissance |
| *Lactobacillus fermentum LMG* | 3,287 | 0,799 | 0,1423 | ns | 0,0015 | ** | Pas d'effet sur la croissance |
| *Lactobacillus paracasei ATCC 25302* | 3,987 | 0,524 | <0,0001 | **** | 0,4258 | ns | SPM (marqué) |
| *Lactobacillus paracasei BL23* | 2,682 | 0,482 | >0,9999 | ns | <0,0001 | **** | Pas d'effet sur la croissance |
| *Lactobacillus paracasei Shirota* | 3,407 | 0,779 | <0,0001 | **** | 0,0984 | ns | SPM (marqué) |
| *Lactobacillus delbrueckii spp.bulgaricus ATCC 11842* | 2,540 | 0,599 | >0,9999 | ns | <0,0001 | **** | Pas d'effet sur la croissance |
| *Lactobacillus casei L919* | 4,150 | 0,712 | <0,0001 | **** | 0,2966 | ns | SPM (marqué) |
| *Lactobacillus rhamnosus L900* | 3,920 | 0,572 | <0,0001 | **** | >0,9999 | ns | SPM (marqué) |

(suite)

| | Tailles des larves à J7 (mm) | | Analyse statistique | | | | Interprétation du test qualitatif (niveau quantitatif) |
|---|---|---|---|---|---|---|---|
| | Moyenne (n>60, mm) | Ecart-type | Comparaison/animaux axéniques | | Comparaison/L. *plantarum WJL* | | |
| | | | Valeur de p (1) | Signification statistique | Valeur de p (1) | Signification statistique | |
| *Lactobacillus rhamnosus L908* | 4,112 | 0,576 | <0,0001 | **** | 0,1227 | ns | SPM (marqué) |
| *Lactobacillus rhamnosus GG* | 4,036 | 0,488 | <0,0001 | **** | 0,5272 | ns | SPM (marqué) |
| | | | *Significativement supérieur à la valeur de références | | *Significativement inférieur à la valeur de références | | SPM = Souche promotrice de croissance |
| (1) Test statistique utilisé : test Mann-Whitney. | | | | | | | |

**Indications sur les critères de validation du test phénotypique "croissance larvaire" :**

La souche doit remplir au minimum le critère 1 pour être validée et l'ampleur de son activité peut être qualifiée selon le critère 1+2.

Critère 1 qualitatif = souche promotrice de croissance par rapport à la condition axénique :

Un test statistique est appliqué à l'ensemble des valeurs de longueur des individus associés par rapport aux valeurs des individus axéniques (60 larves au minimum par condition). Une valeur p inférieure à 0,001 (test Mann-Whitney) qualifie la souche de "promotrice de croissance". Dans le cas présent les souches suivantes sont qualifiées de promotrice de croissance :

*Lactobacillus plantarum WJL*
*Lactobacillus plantarum NIZO2877*
*Lactobacillus casei ATCC 393*
*Lactobacillus fermentum ATCC 9338*
*Lactobacillus paracasei ATCC 25302*
*Lactobacillus paracasei Shirota*
*Lactobacillus casei L919*
*Lactobacillus rhamnosus L900*
*Lactobacillus rhamnosus L908*
*Lactobacillus rhamnosus GG*

Les souches suivantes ne présentent pas d'effet promoteur de croissance :

*Lactobacillus fermentum KLD*
*Lactobacillus fermentum LMG*
*Lactobacillus paracasei BL23*
*Lactobacillus delbrueckii spp.bulgaricus ATCC 11842*

Critère 2 quantitatif = ampleur de l'effet de promotion de croissance (avec le critère 1 validé préalablement) :

Un test statistique est appliqué à l'ensemble des valeurs de longueur des individus associés à la ou les souches testées par rapport aux valeurs des individus mono-associés à la souche *L. plantarum WJL* (60 larves au minimum par condition).
Trois cas de figures se présentent :
La valeur p du test statistique est supérieure à 0,001 (test Mann-Whitney), la souche sera qualifiée d'un effet "marqué" sur la croissance larvaire (effet similaire à la souche L. *plantarum WJL*).

Dans l'exemple présent les souches suivantes ont un effet marqué :

*Lactobacillus casei ATCC 393*
*Lactobacillus fermentum ATCC 9338*
*Lactobacillus paracasei ATCC 25302*
*Lactobacillus paracasei Shirota*
*Lactobacillus casei L919*
*Lactobacillus rhamnosus L900*
*Lactobacillus rhamnosus L908*
*Lactobacillus rhamnosus GG*
*Lactobacillus plantarum WJL*

La valeur p du test statistique est inférieure à 0,001 (test Mann-Whitney), la souche sera qualifiée d'un effet "intermédiaire" sur la croissance larvaire si la taille moyenne des individus est inférieure à celles des individus associés à *L. plantarum WJL* (cas de la souche *L. plantarum NIZO* 2877 dans l'exemple présenté). La souche sera qualifiée d'un effet "fort" si la taille moyenne des individus est supérieure à celles des individus associés à *L. plantarum WJL* (cas non identifié dans l'exemple présent).

**Analyse de l'exemple de test phénotypique "croissance larvaire"** :

- 14 souches de *Lactobacillus* ont été testées (Tableaux 1) afin de déterminer leur potentiel de promotion de croissance à l'aide du test de "croissance larvaire" chez *Drosophila melanogaster.*
- Ces souches sont disponibles à l'ATCC, à l'Institut Pasteur de Paris, à l'Institut Pasteur de Lille ou publié dans la littérature scientifique et disponible auprès des chercheurs référents des publications mentionnées (Tableau 2).

Tableau 2 :

| | Collection publique et/ou publication avec séquence du génome |
|---|---|
| *Lactobacillus plantarum WJL* | Kim et al., Genome Announc. 21 novembre 2013, 1(6).Pil : e00937-13 |
| *Lactobacillus plantarum NIZO2877* | NIZO (2877) |
| *Lactobacillus casei ATCC 393* | ATCC (393) |
| *Lactobacillus fermentum ATCC 9338* | ATCC (9338) |
| *Lactobacillus fermentum KLD* | Institut Pasteur de Lille (A5.20) |
| *Lactobacillus fermentum LMG* | Institut Pasteur de Lille (A5.16) |
| *Lactobacillus paracasei ATCC 25302* | ATCC (25302) |
| *Lactobacillus paracasei BL23* | Institut Pasteur de Lille (A3.6) et Mazé et al., J. Bacteriol., Mai 2010; 192(10) : 2647-8 |
| *Lactobacillus paracasei Shirota* | Institut Pasteur de Lille (A3.5) |
| *Lactobacillus delbrueckii spp.bulgaricus* | ATCC (11842) et van de Guchte M, et al., Proc.Natl Acad Sci USA 13 juin 2006 |
| *Lactobacillus casei L919* | Koryszewska-Baginska A et al., Genome Announc, 26 septembre 2013 |
| *Lactobacillus rhamnosus L900* | Aleksandrzak-Piekarczyk T et al. Genome Announc , 15 août 2013 |
| *Lactobacillus rhamnosus L908* | Koryszewska-Baginska A et al. Genome Announc, 20 février 2014 |
| *Lactobacillus rhamnosus GG* | ATCC (53103) et Kankainen M et al., Proc Natl Acad Sci USA, 6 octobre 2009 |

- La taille d'un minimum de 60 individus par condition est étudiée au jour 7 après association avec la souche bactérienne à tester, puis élevage des individus juvéniles en milieu nutritionnel carencé en levure. Le tableau 1 présente la moyenne et l'écart-type de ces jeux de données.
- L'analyse statistique de ces résultats permet de qualifier selon le critère 1 du "test de croissance larvaire" 10 souches de différentes espèces de *Lactobacilles.* Cette sélection met en lumière un effet fonctionnel strictement souche spécifique et pas nécessairement lié à une espèce donnée de Lactobacille.

- L'analyse statistique des résultats permet selon le critère 2, de qualifier d'"intermédiaire" l'effet de la souche *L. plantarum NIZO 2877* alors que les 9 autres souches promotrices de croissance présentent des effets "marqués".

**Exemple 2 : Description d'une souche à effet fort sur le modèle Drosophile**

Le test Drosophile de l'exemple 1 est reproduit avec 3 souches de bactéries :

- *Lactobacillus plantarum* WJL
- *Lactobacillus plantarum* NIZO2877
- *Lactobacillus plantarum* G821

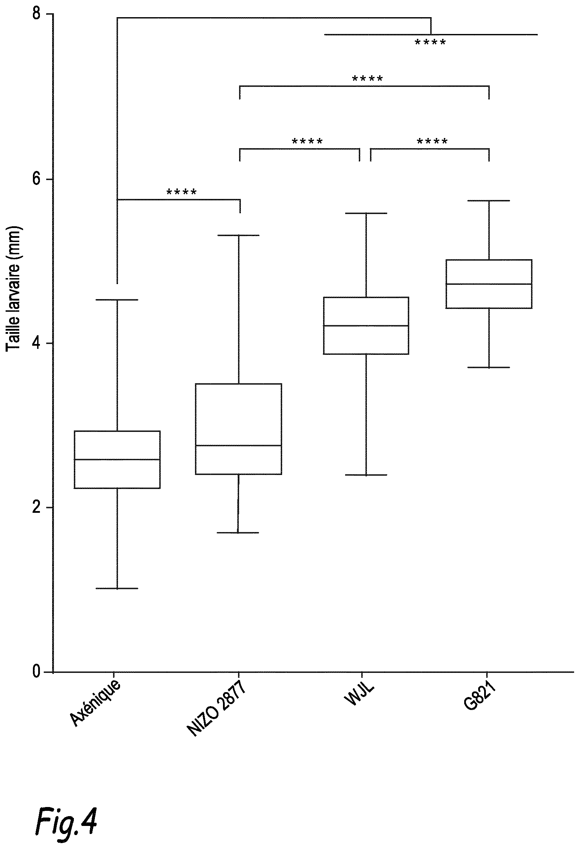

L'analyse des résultats (voir Figure 4) montre les effets suivants :

Axénique versus NIZO 2877 p<0,001 (***)
NIZO 2877 versus WJL p<0,0001 (****)
G821 versus WJL p<0,0001 (****)
Axénique versus WJL ou G821 p<0,0001 (****)

Conclusion :

- *Lactobacillus plantarum* WJL : effet marqué
- *Lactobacillus plantarum* NIZO2877 : effet intermédiaire
- *Lactobacillus plantarum* G821 : effet fort.

**Exemple 3 : Criblage fonctionnel de bactéries sur souris - analyse phénotypique** :

Etude chez la souris de l'effet promoteur de croissance juvénile de deux souches à effet "intermédiaire" et "marqué" *(L. plantarum NIZO2877* et *L. plantarum WJL,* respectivement) identifié à l'aide du test "croissance larvaire" de l'exemple 1.

La descendance male (minimum 15 individus) de trois groupes d'individus issus d'une même colonie de souris axénique a été étudiée, le premier groupe consiste en des juvéniles axéniques (groupe GF), le deuxième en des juvéniles issus de parents mono-associés avec la souche *L. plantarum WJL* (groupe WJL), et le troisième en des juvéniles issus de parents mono-associés avec la souche *L. plantarum NIZO2877* (groupe NIZO 2877). Les parents et juvéniles sont élevés en milieu nutritionnel conventionnel jusqu'au sevrage des juvéniles (J21), puis les juvéniles sevrés sont élevés en milieu nutritionnel carencé en protéines et en lipides (8% et 2.5% respectivement) jusqu'à J56.

Milieu nutritionnel conventionnel : 23% protéines et 5% lipides
Milieu nutritionnel carencé : 8% protéines et 2,5% lipides

Trois paramètres illustrant la croissance juvénile de ces individus ont été étudiés : (1) la prise de taille pendant une période de 28 jours suivant le sevrage (de J28 à J56), (2) la taille des fémurs d'un lot d'individus (au minimum 9 individus) représentatif de la population testée à J56 et enfin (3) le taux sérique à J56 du facteur de croissance IGF-1 chez au moins 8 individus.

Les analyses statistiques ont été réalisées en utilisant le test de Tukey en utilisant le logiciel GraphPad (GraphPad Prism 5.04, San Diego, USA) ; valeurs de p < 0.05 sont considérées significatives.

(1) Prise de taille :

Les souris ont été anesthésiées par brève exposition à l'isoflurane pour permettre la mesure de la taille des souris (du museau à la base de la queue) à J28 et J56.

Tableau 3 : taux de croissance (prise de taille par jour en cm de J28 à J56) – Nb = nombre de souris

| GF | | | WJL | | | NIZO 2877 | | |
|---|---|---|---|---|---|---|---|---|
| Moyenne | Ecart-type | Nb | Moyenne | Ecart-type | Nb | Moyenne | Ecart-type | Nb |
| 0,007321 | 0,009878 | 15 | 0,031114 | 0,009376 | 17 | 0,016429 | 0,011071 | 25 |

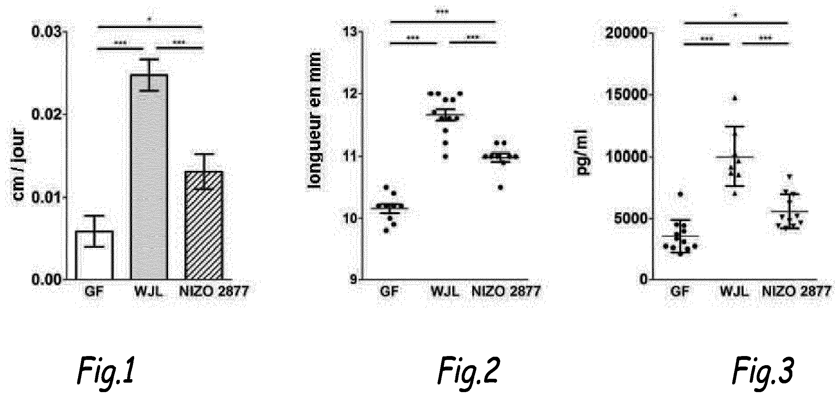

Ces données sont réunies sur la Figure 1.

(2) Taille des fémurs :

Les souris sont sacrifiées à J56, un fémur est prélevé, libéré du muscle et sa longueur est mesurée au caliper Vernier.

Tableau 4 : Longueur des fémurs en mm à J56 chez les mâles

| GF | WJL | NIZO 2877 |
|---|---|---|
| 10,2 | 11,2 | 10,9 |
| 10,2 | 11,6 | 11 |
| 10,5 | 11,9 | 10,5 |
| 9,9 | 11,6 | 11,2 |
| 10,4 | 12 | 11 |
| 10 | 11,4 | 11 |
| 10,2 | 12 | 11 |
| 10, 2 | 11,7 | 11,2 |
| 9,8 | 12 | 11 |
| | 11,9 | |
| | 11,6 | |
| | 11 | |

Ces données sont réunies sur la Figure 2.

(3) Titres en IGF-1 dans le sérum :

Les titres en IGF-1 sont mesurés sur le sérum obtenu à partir du sang des souris sacrifiées à J56. La mesure est effectuée sur du sérum dilué (1:25) en utilisant le kit ELISA Ready-Set-Go (eBioscience, USA), en suivant les instructions du fabricant.

Tableau 5 : Titre sérique en IGF-1 en pg/ml (double mesure à partir des plaques ELISA)

| GF | | WJL | | NIZO 2877 | |
|---|---|---|---|---|---|
| 4526,75 | 4343,75 | 8823,25 | 8606,5 | 7291,25 | 6998,75 |
| 2825,5 | 2614,75 | 9258,5 | 9122,25 | 5128 | 5102 |
| 2667,5 | 2773 | 8471,25 | 8660,5 | 4343,75 | 4396 |
| 3455,25 | 3219,5 | 7025,25 | 7078,25 | 5232,75 | 5180,5 |
| 7131,5 | 6839,5 | 11063 | 9557,3 | 8471,25 | 8282,5 |
| 2164,2 | 2057,55 | 15125 | 14409 | 4866,5 | 4761,75 |
| 3140,75 | 3035,75 | 10023 | 9372 | 6363,25 | 6152,25 |
| 2509 | 2456,15 | 11735 | 12026 | 7211,25 | 6680,5 |
| 2588,25 | 2614,75 | | | 4213,25 | 4161 |
| 4432,5 | 4601,3 | | | 4770,5 | 4517 |
| 4013,3 | 3930 | | | 4940,3 | 3846,5 |
| 3597,3 | 3846,5 | | | | |

Ces données sont réunies sur la Figure 3.

Les résultats illustrent un effet "marqué" de la souche *L. plantarum WJL* sur le taux de croissance (valeur p <0.001 par rapport à la condition axénique) et un effet "intermédiaire" de la souche *L. plantarum NIZO2877* (valeur p<0.05 par rapport à la condition axénique et valeur p<0.001 par rapport à la condition *L. plantarum WJL*). Ces effets sont confirmés avec le paramètre "taux d'IGF-1" (mêmes gammes de valeurs p des différents tests), puis renforcés avec le paramètre "taille des fémurs" (valeur p<0.001 entre les conditions axénique et *L. plantarum NIZO 2877*)

L'ensemble de ces résultats démontre par la preuve scientifique l'effet promoteur de croissance juvénile de certaines souches de *Lactobacilles* répondant également positivement au test de "croissance larvaire".

L'homme du métier pourra se référer aux dépôts de souches commerciales auxquels il est fait référence ici.

## Revendications

1. Composition comprenant au moins une souche de *Lactobacillus* à tropisme intestinal, choisie parmi les espèces *Lactobacillus plantarum* ou *Lactobacillus fermentum,* pour une utilisation visant à favoriser la croissance juvénile dans le traitement de la malnutrition des mammifères, dans laquelle la souche de *Lactobacillus* répond positivement à au moins l'un des tests A), B), C) et D) suivants :

   Test A)

   - on dispose d'un lot d'embryons de parents *Drosophila melanogaster* axéniques ;
   - à J1 : on répartit ces embryons en au moins 2 groupes (40 embryons, réalisé en triplicat soit 120 embryons par groupe) sur un milieu nutritionnel comprenant de la farine de maïs, de l'agar et de l'eau, carencé en levure, l'un des groupes d'embryons est en outre inoculé par une suspension d'environ $10^8$ CFU de la bactérie à tester dans un tampon salin, ce qui forme le groupe monoxénique, l'autre groupe étant le groupe axénique, on conduit l'élevage des deux groupes à environ 25°C jusqu'à J7 ;
   - à J7 : on récupère au minimum 60 larves de drosophiles de chaque groupe, on leur applique un choc thermique;
   - on détermine la moyenne de la longueur des larves de chacun des groupes, et on compare les moyennes obtenues ;
   - la souche de bactérie testée est considérée comme répondant positivement au test si la moyenne de la longueur des larves du groupe monoxénique est supérieure à la moyenne de la longueur des larves du groupe axénique avec une valeur de p inférieure ou égale à 0,001, de préférence inférieure ou égale à 0,0001 dans le test statistique de Mann-Whitney, réalisé sur l'ensemble du jeu de données des tailles des larves des deux groupes

   Test B)

   - à partir d'une même lignée de souris on établit une lignée de souris parentales axéniques et une lignée de souris parentales monoxéniques (associées à la bactérie à tester), et l'on produit des juvéniles qui sont élevées avec les parents en milieu nutritionnel conventionnel jusqu'à leur sevrage (J21)
   - à J21 : on dispose de 8 juvéniles sevrées issues de chacune de ces deux lignées, formant le groupe monoxénique et le groupe axénique, et on les élève en régime nutritionnel carencé en protéines (8%) et en lipides (2,5%);
   - à J 56 : on détermine la moyenne des tailles des souris pour chaque groupe considéré par la mesure de l'extrémité du museau à la base de la queue de chaque individu, ou l'on prélève les fémurs des individus de chaque groupe et l'on mesure ces derniers ;
   - la souche de bactérie lactique étant considérée comme répondant positivement au test si la taille moyenne des individus ou des fémurs du groupe monoxénique est supérieur à la taille moyenne des individus ou des fémurs, du groupe axénique, avec une valeur de p inférieure ou égale à 0,05 dans le test statistique de Tukey ;

   Test C)

   - à partir d'une même lignée de souris axéniques on établit une lignée de souris parentales axéniques et une lignée de souris parentales monoxéniques (associées à la bactérie à tester), et l'on produit des juvéniles qui sont élevées avec les parents en milieu nutritionnel conventionnel jusqu'à leur sevrage (J21),
   - à J21 : on dispose de 8 juvéniles sevrées issues de chacune de ces deux lignées, formant le groupe monoxénique et le groupe axénique, et on les élève en régime nutritionnel carencé en protéines (8%) et en lipides (2,5%);
- à J 56 : on prélève du sang des juvéniles de chaque groupe et on détermine le taux sérique moyen d'IGF-1 pour chaque groupe ;
- la souche de bactérie lactique étant considérée comme répondant positivement au test si le taux sérique moyen d'IGF-1 du groupe monoxénique est supérieur au taux sérique moyen du groupe axénique avec une valeur de p inférieure ou égale à 0,05 dans le test statistique de Tukey

Test D)

- on dispose d'un lot d'embryons de parents *Drosophila melanogaster* axéniques ;
- à J1 : on répartit ces embryons en au moins 2 groupes (40 embryons, réalisé en triplicat soit 120 embryons par groupe) sur un milieu nutritionnel comprenant de la farine de maïs, de l'agar et de l'eau, carencé en levure, l'un des groupes d'embryons est inoculé par une suspension d'environ $10^8$ CFU de ladite bactérie dans un tampon salin, ce qui forme le groupe monoxénique test, l'autre groupe étant inoculé par une suspension d'environ $10^8$ CFU de bactérie de référence *L. plantarum* WJL ou *L. fermentum* ATCC 9338, dans un tampon salin, formant le groupe monoxénique de référence, on conduit l'élevage des deux groupes à environ 25°C jusqu'à J7 ;
- à J7 : on récupère au minimum 60 larves de drosophiles de chaque groupe, on leur applique un choc thermique;
- on détermine la moyenne de la longueur des larves de chacun des groupes, et on compare les moyennes obtenues ;
- ladite souche de bactérie ayant, par rapport à la bactérie de référence, un effet fort avec une moyenne de la longueur des larves du groupe monoxénique test avec cette souche de bactérie significativement supérieure à la moyenne pour le groupe monoxénique avec la souche de référence, avec une valeur de p est inférieure ou égale à 0,001, de préférence à 0,0001 dans le test statistique de Mann-Whitney, réalisé sur l'ensemble du jeu de données des tailles des larves des deux groupes.

2. Composition pour son utilisation selon la revendication 1, dans laquelle la souche de *Lactobacillus* est choisie parmi *L. plantarum* WJL, *L. plantarum* G821, et *L. fermentum* ATCC9338.

3. Composition pour son utilisation selon la revendication 1, comprenant dans ledit test A) une souche de bactérie qui, par rapport à la souche *L. plantarum* WJL, dans ce même test sur drosophile, a un effet fort avec une moyenne de la longueur des larves du groupe monoxénique avec cette souche de bactérie significativement supérieure à la moyenne pour le groupe monoxénique avec la souche WJL, lorsque la valeur de p est inférieure ou égale à 0,001, de préférence à 0,0001 dans le test statistique de Mann-Whitney, réalisé sur l'ensemble du jeu de données des tailles des larves des deux groupes.

4. Composition pour son utilisation selon la revendication 1, comprenant dans ledit test B) une souche de bactérie qui, par rapport à la souche *L. plantarum* WJL, dans ce même test sur souris, a un effet fort avec une moyenne de la taille des souris ou de leurs du groupe monoxénique avec cette souche de bactérie significativement supérieure à la moyenne pour du groupe monoxénique avec la souche WJL, lorsque la valeur de p est inférieure ou égale à 0,05 dans le test statistique de Tukey.

5. Composition pour son utilisation selon la revendication 1, comprenant dans ledit test C) une souche de bactérie qui, par rapport à la souche *L. plantarum* WJL, dans ce même test sur souris, a un effet fort avec une moyenne de taux sérique d'IGF-1 du groupe monoxénique avec cette souche de bactérie significativement supérieure à la moyenne pour le groupe monoxénique avec la souche WJL, lorsque la valeur de p est inférieure ou égale à 0,05 dans le test statistique de Tukey.

6. Composition pour son utilisation selon l'une quelconque des revendications 1 à 5, où la malnutrition des mammifères est **caractérisée par** une carence protéique.

7. Composition pour son utilisation selon l'une quelconque des revendications précédentes, où la malnutrition des mammifères est **caractérisée par** un taux sérique d'IGF-1 inférieur à la normale.

8. Composition pour son utilisation selon l'une quelconque des revendications précédentes, dans laquelle la bactérie est un *Lactobacillus plantarum*.

9. Composition pour son utilisation selon la revendication 8, dans laquelle la bactérie est un *Lactobacillus plantarum* G821 déposée à la Collection Nationale de Culture de Microorganismes (Institut Pasteur) sous le numéro d'enregistrement CNCM I-4979.

10. Composition pour son utilisation selon l'une quelconque des revendications précédentes, contenant de $10^5$ à $10^{12}$ CFU de *Lactobacillus* répondant positivement au test, par gramme de composition.

11. Méthode de criblage de bactéries capables de favoriser la croissance juvénile en cas de malnutrition des mammifères, notamment homme, animaux de rente, animaux de compagnie et animaux de sport, dans laquelle la souche de bactérie est soumise au test suivant :

   - on dispose d'un lot d'embryons de parents de *Drosophila melanogaster* axéniques ;
   - on place ces embryons en au moins 2 groupes sur un milieu nutritionnel comprenant un milieu nutritif pour drosophiles carencé en levure, l'un des groupes d'embryons est en outre inoculé par une suspension de la bactérie à tester, ce qui forme le groupe des individus monoxéniques ; l'autre groupe forme le groupe axénique,
   - on conduit l'élevage à température appropriée,
   - au terme d'une période d'élevage appropriée, on récupère, de préférence en nombre égal, des larves de drosophiles de chaque groupe (larves axéniques et larves monoxéniques),
   - on détermine la moyenne de la longueur des larves de chacun des groupes, et on compare les moyennes obtenues ;
   - la souche de bactérie testée est considérée comme répondant positivement au test si la moyenne de la longueur des larves du groupe monoxénique est supérieure à la moyenne de la longueur des larves du groupe axénique avec une valeur de p inférieure ou égale à 0,001, de préférence inférieure ou égale à 0,0001 dans le test de Mann-Whitney, réalisé sur l'ensemble du jeu de données.

12. Méthode de criblage de bactéries capables de favoriser la croissance juvénile en cas de malnutrition, dans laquelle la souche de bactérie est soumise au test suivant :

   - on dispose de juvéniles issues de deux lignées issues d'une même souche de souris, à savoir une lignée de souris axéniques et une lignée de souris monoxéniques,
   - on les élève en régime nutritionnel carencé en protéines et de préférence aussi en lipides ;
   - au terme d'une période d'élevage appropriée, on détermine la moyenne des valeurs d'un ou plusieurs paramètres de chaque groupe, ces paramètres étant liés au taux d'IGF-1 sérique ou à la croissance,
   - la souche de bactérie lactique est considérée comme répondant positivement au test si le taux moyen d'IGF-1 du groupe monoxénique est supérieur au taux moyen du groupe axénique avec une valeur de p inférieure ou égale à 0,05 dans le test statistique Tukey.

13. Composition, culture pure ou isolat comprenant la bactérie *Lactobacillus plantarum* G821 déposée à la Collection Nationale de Culture de Microorganismes (Institut Pasteur) sous le numéro d'enregistrement CNCM I-4979.

## Patentansprüche

1. Zusammensetzung, die mindestens einen *Lactobacillus-Stamm* mit Darmtropismus umfasst, der aus den Spezies *Lactobacillus plantarum* oder *Lactobacillus fermentum* ausgewählt ist, für eine Verwendung, die darauf abzielt, das Jugendwachstum bei der Behandlung von Mangelernährung bei Säugetieren zu begünstigen, wobei der *Lactobacillus-Stamm* positiv auf mindestens einen der folgenden Tests A), B), C) und D) reagiert:

   Test A)

   - Verfügen über eine Partie Embryonen von axenischen *Drosophila melanogaster* Eltern;
   - am T1: Verteilen dieser Embryonen auf mindestens 2 Gruppen (40 Embryonen, dreifach ausgeführt, das heißt 120 Embryonen pro Gruppe) auf einem Nährmedium, das Maismehl, Agar und Wasser umfasst, mit Mangel an Hefe, wobei eine der Embryonengruppen ferner mit einer Suspension von ungefähr $10^8$ KBE der zu testenden Bakterie in einem salzhaltigen Puffer geimpft wird, wodurch die monoxenische Gruppe gebildet wird, wobei die andere Gruppe die axenische Gruppe ist, die Aufzucht der zwei Gruppen wird bei ungefähr 25 °C bis T7 fortgesetzt;
   - am T7: Sammeln von mindestens 60 Drosophilia-Larven von jeder Gruppe und Anwenden eines Thermoschocks darauf;

- Bestimmen des Mittels der Länge der Larven jeder Gruppe und Vergleichen der erhaltenen Mittel;
- der getestete Bakterienstamm wird als positiv auf den Test reagierend betrachtet, wenn das Mittel der Länge der Larven der monoxenischen Gruppe im statistischen Mann-Whitney-Test, der auf dem gesamten Datensatz der Größen der Larven der zwei Gruppen ausgeführt wird, mit einem p-Wert von kleiner oder gleich 0,001, vorzugsweise kleiner oder gleich 0,0001, größer als das Mittel der Länge der Larven der axenischen Gruppe ist

Test B)

- ausgehend von einer gleichen Linie axenischer Mäuse, Erstellen einer Linie axenischer Elternmäuse und einer Linie monoxenischer Elternmäuse (die mit der zu testenden Bakterie verbunden sind) und Produzieren von Jungtieren, die bis zu ihrem Abstillen (T21) mit den Eltern im herkömmlichen Nährmedium aufgezogen werden,
- am T21: Verfügen über 8 abgestillte Jungtiere, die von jeder dieser zwei Linien stammen, die die monoxenische Gruppe und die axenische Gruppe bilden, und ihre Aufzucht mit einer Ernährung mit Mangel an Proteinen (8 %) und an Lipiden (2,5 %);
- am T56: Bestimmen des Mittels der Größen der Mäuse für jede betrachtete Gruppe durch die Messung vom Ende der Schnauze bis zur Basis des Schwanzes von jedem Individuum oder Entnehmen der Femora der Individuen jeder Gruppe und Messen dieser letzteren;
- wobei der Milchsäurebakterienstamm als positiv auf den Test reagierend betrachtet wird, wenn die mittlere Größe der Individuen oder der Femora der monoxenischen Gruppe beim statistischen Tukey-Test mit einem p-Wert von kleiner oder gleich 0,05 größer als die mittlere Größe der Individuen oder der Femora der axenischen Gruppe ist;

Test C)

- ausgehend von einer gleichen Linie axenischer Mäuse, Erstellen einer Linie axenischer Elternmäuse und einer Linie monoxenischer Elternmäuse (die mit der zu testenden Bakterie verbunden sind) und Produzieren von Jungtieren, die bis zu ihrem Abstillen (T21) mit den Eltern im herkömmlichen Nährmedium aufgezogen werden,
- am T21: Verfügen über 8 abgestillte Jungtiere, die von jeder dieser zwei Linien stammen, die die monoxenische Gruppe und die axenische Gruppe bilden, und ihre Aufzucht mit einer Ernährung mit Mangel an Proteinen (8 %) und an Lipiden (2,5 %);
- am T56: Entnehmen von Blut bei den Jungtieren von jeder Gruppe und Bestimmen des mittleren Serumspiegels von IGF-1 für jede Gruppe;
- wobei der Milchsäurebakterienstamm als positiv auf den Test reagierend betrachtet wird, wenn der mittlere Serumspiegel von IGF-1 der monoxenischen Gruppe im statistischen Tukey-Test mit einem p-Wert von kleiner oder gleich 0,05 höher als der mittlere Serumspiegel der axenischen Gruppe ist

Test D)

- Verfügen über eine Partie Embryonen von axenischen *Drosophila melanogaster* Eltern;
- am T1: Verteilen dieser Embryonen auf mindestens 2 Gruppen (40 Embryonen, dreifach ausgeführt, das heißt 120 Embryonen pro Gruppe) auf einem Nährmedium, das Maismehl, Agar und Wasser umfasst, mit Mangel an Hefe, wobei eine der Embryonengruppen ferner mit einer Suspension von ungefähr $10^8$ KBE der Bakterie in einem salzhaltigen Puffer geimpft wird, wodurch die monoxenische Testgruppe gebildet wird, wobei die andere Gruppe mit einer Suspension von ungefähr $10^8$ KBE Bezugsbakterie *L. plantarum* WJL oder *L. fermentum* ATCC 9338 in einem salzhaltigen Puffer geimpft wird, wodurch die monoxenische Bezugsgruppe gebildet wird, die Aufzucht wird bei ungefähr 25 °C bis T7 fortgesetzt;
- am T7: Sammeln von mindestens 60 Drosophilia-Larven von jeder Gruppe und Anwenden eines Thermoschocks darauf;
- Bestimmen des Mittels der Länge der Larven jeder der Gruppen und Vergleichen der erhaltenen Mittel;
- wobei der erhaltene Bakterienstamm in Bezug auf die Bezugsbakterie mit einem Mittel der Länge der Larven der monoxenischen Testgruppe mit diesem Bakterienstamm, das wesentlich höher als das Mittel für die monoxenische Gruppe mit dem Bezugsstamm ist, mit einem p-Wert von kleiner oder gleich 0,001, vorzugsweise 0,0001 im statistischen Mann-Whitney-Test, der auf dem gesamten Datensatz der Größen der Larven der zwei Gruppen durchgeführt wird, eine starke Wirkung aufweist.

2. Zusammensetzung zur Verwendung nach Anspruch 1, wobei der *Lactobacillus-Stamm* aus *L. plantarum* WJL, *L. plantarum* G821 und L. *fermentum* ATCC9338 ausgewählt ist.

3. Zusammensetzung zur Verwendung nach Anspruch 1, die im Test A) einen Bakterienstamm umfasst, der in Bezug auf den Stamm *L. plantarum* WJL in diesem gleichen Test auf Drosophilia mit einem Mittel der Länge der Larven der monoxenischen Testgruppe mit diesem Bakterienstamm, das wesentlich größer als das Mittel für die monoxenische Gruppe mit dem Stamm WJL ist, eine starke Wirkung aufweist, wenn im statistischen Mann-Whitney-Test, der auf dem gesamten Datensatz der Größen der Larven der zwei Gruppen durchgeführt wird, der p-Wert kleiner oder gleich 0,001, vorzugsweise 0,0001, ist.

4. Zusammensetzung zur Verwendung nach Anspruch 1, die im Test B) einen Bakterienstamm aufweist, der in Bezug auf den Stamm *L. plantarum* WJL in diesem gleichen Test auf Mäusen mit einem Mittel der Größe der Mäuse der monoxenischen Gruppe mit diesem Bakterienstamm oder ihrer Femora, das wesentlich größer als das Mittel für die monoxenische Gruppe mit dem Stamm WJL ist, eine starke Wirkung aufweist, wenn im statistischen Tukey-Test der p-Wert kleiner oder gleich 0,05 ist.

5. Zusammensetzung zur Verwendung nach Anspruch 1, die im Test C) einen Bakterienstamm umfasst, der in Bezug auf den Stamm *L. plantarum* WJL in diesem gleichen Test auf Mäusen mit einem Mittel des Serumspiegels von IGF-1 der monoxenischen Gruppe mit diesem Bakterienstamm, das wesentlich größer ist als das Mittel für die monoxenische Gruppe mit dem Stamm WJL, eine starke Wirkung aufweist, wenn im statistischen Tukey-Test der p-Wert kleiner oder gleich 0,05 ist.

6. Zusammensetzung zur Verwendung nach einem der Ansprüche 1 bis 5, wobei die Mangelernährung der Säugetiere durch einen Proteinmangel gekennzeichnet ist.

7. Zusammensetzung zur Verwendung nach einem der vorhergehenden Ansprüche, wobei die Mangelernährung der Säugetiere durch einen Serumspiegel von IGF-1 gekennzeichnet ist, der niedriger als normal ist.

8. Zusammensetzung zur Verwendung nach einem der vorhergehenden Ansprüche, wobei die Bakterie ein *Lactobacillus plantarum* ist.

9. Zusammensetzung zur Verwendung nach Anspruch 8, wobei die Bakterie ein *Lactobacillus plantarum* G821 ist, der bei der Collection Nationale de Culture de Microorganismes (Institut Pasteur) unter der Hinterlegungsnummer CNCM I-4979 hinterlegt ist.

10. Zusammensetzung zur Verwendung nach einem der vorhergehenden Ansprüche, die pro Gramm Zusammensetzung $10^5$ bis $10^{12}$ KBE Lactobacillus enthält, der positiv auf den Test reagiert.

11. Verfahren zum Screening von Bakterien, die in der Lage sind, das Jugendwachstum bei Mangelernährung von Säugetieren, insbesondere beim Menschen, bei Nutztieren, Haustieren und Sporttieren, zu begünstigen, wobei der Bakterienstamm dem folgenden Test unterzogen wird:

   - Verfügen über eine Partie Embryonen von axenischen *Drosophila melanogaster* Eltern;
   - Platzieren dieser Embryonen in mindestens 2 Gruppen auf einem Nährmedium, das Ernährungsmedium mit Hefemangel für Drosophilia umfasst, wobei eine der Gruppen von Embryonen ferner mit einer Suspension der zu testenden Bakterie geimpft wird, woraus die Gruppe monoxenischer Individuen gebildet wird; wobei die andere Gruppe die axenische Gruppe bildet,
   - Durchführen der Aufzucht bei geeigneter Temperatur,
   - am Ende einer geeigneten Aufzuchtperiode, Entnehmen der Drosophilia-Larven jeder Gruppe (axenische Larven und monoxenische Larven), vorzugsweise in gleicher Anzahl,
   - Bestimmen des Mittels der Länge der Larven von jeder der Gruppen und Vergleichen der erhaltenen Mittel;
   - der getestete Bakterienstamm wird als positiv auf den Test reagierend betrachtet, wenn das Mittel der Länge der Larven der monoxenischen Gruppe im Mann-Whitney-Test, der auf dem gesamten Datensatz ausgeführt wird, mit einem p-Wert von kleiner oder gleich 0,001, vorzugsweise kleiner oder gleich 0,0001, größer als das Mittel der Länge der Larven der axenischen Gruppe ist.

12. Verfahren zum Screening von Bakterien, die in der Lage sind, das Jugendwachstum bei Mangelernährung zu begünstigen, wobei der Bakterienstamm dem folgenden Test unterzogen wird:

- Verfügen über Jungtiere, die aus zwei Linien stammen, die von einem gleichen Mäusestamm stammen, das heißt einer axenischen Mäuselinie und einer monoxenischen Mäuselinie,
- ihre Aufzucht mit einer Ernährung mit Mangel an Proteinen und vorzugsweise auch an Lipiden;
- nach einer geeigneten Aufzuchtperiode, Bestimmen des Mittels der Werte von einem oder mehreren Parametern jeder Gruppe, wobei diese Parameter mit dem IGF-1-Serumwert oder mit dem Wachstum verbunden sind,
- der Milchsäurebakterienstamm als positiv auf den Test reagierend betrachtet wird, wenn der mittlere IGF-1-Spiegel der monoxenischen Gruppe im statistischen Tukey-Test mit einem p-Wert von kleiner oder gleich 0,05 höher als der mittlere Spiegel der axenischen Gruppe ist.

13. Zusammensetzung, Reinkultur oder Isolat, die bzw. das die Bakterie *Lactobacillus plantarum* G821 umfasst, die bei der Collection Nationale de Culture de Microorganismes (Institut Pasteur) unter der Hinterlegungsnummer CNCM I-4979 hinterlegt ist.

## Claims

1. A composition comprising at least a *Lactobacillus* strain with intestinal tropism selected from the species *Lactobacillus plantarum* or *Lactobacillus fermentum,* for use in promoting juvenile growth in the treatment of malnutrition in mammals, wherein the *Lactobacillus* strain responds positively to at least one of the following tests A), B), C) and D):

    Test A)

    - a batch of embryos from axenic *Drosophila melanogaster* parents is available;
    - on D1: these embryos are distributed into at least 2 groups (40 embryos, performed in triplicate, i.e., 120 embryos per group) on a nutritional medium comprising corn flour, agar and water, deficient in yeast, one of the embryo groups is further inoculated with a suspension of about $10^8$ CFU of the bacterium to be tested in a saline buffer, which forms the monoxenic group, the other group being the axenic group, the rearing of both groups is conducted at about 25°C up to D7;
    - on D7: at least 60 *Drosophila* larvae from each group are recovered and heat-shocked;
    - the mean length of the larvae of each group is determined, and the means obtained are compared;
    - the tested bacterial strain is considered to respond positively to the test if the mean of the length of the larvae of the monoxenic group is greater than the mean of the length of the larvae of the axenic group with a p-value less than or equal to 0.001, preferably lower than or equal to 0.0001 in the Mann-Whitney statistical test, carried out on the entire data set of the sizes of the larvae of both groups

    Test B)

    - from the same mouse line, a line of axenic parent mice and a line of monoxenic parent mice (associated with the bacterium to be tested) are established, and juveniles are produced which are reared with the parents in a conventional nutritional medium until they are weaned (D21);
    - on D21: 8 weaned juveniles from each of these two lines, forming the monoxenic group and the axenic group, are reared on a nutritional diet deficient in protein (8%) and lipids (2.5%);
    - on D56: the mean size of the mice for each group considered is determined by measuring from the tip of the snout to the base of the tail of each individual, or the femurs of the individuals of each group are taken and measured;
    - the strain of lactic acid bacteria is considered to respond positively to the test if the mean size of the individuals or femurs of the monoxenic group is greater than the mean size of the individuals or femurs of the axenic group, with a p-value less than or equal to 0.05 in Tukey's statistical test;

    Test C)

    - from the same axenic mouse line, a line of axenic parent mice and a line of monoxenic parent mice (associated with the bacterium to be tested) are established, and juveniles are produced which are reared with the parents in a conventional nutritional medium until they are weaned (D21);
    - on D21: 8 weaned juveniles from each of these two lines, forming the monoxenic group and the axenic group, are reared on a nutritional diet deficient in protein (8%) and lipids (2.5%);
    - on D56: blood is taken from the juveniles of each group and the mean serum IGF-1 level is determined for each group;
- the strain of lactic acid bacteria is considered to respond positively to the test if the mean serum IGF-1 level of the monoxenic group is higher than the mean serum level of the axenic group with a p-value less than or equal to 0.05 in Tukey's statistical test

Test D)

- a batch of embryos from axenic *Drosophila melanogaster* parents is available;
- on D1: these embryos are distributed into at least 2 groups (40 embryos, performed in triplicate, i.e., 120 embryos per group) on a nutritional medium comprising corn flour, agar and water, deficient in yeast, one of the embryo groups is inoculated with a suspension of about $10^8$ CFU of said bacterium in a saline buffer, which forms the monoxenic test group, the other group being inoculated with a suspension of about $10^8$ CFU of the reference bacterium *L. plantarum* WJL or *L. fermentum* ATCC 9338, in a saline buffer, forming the monoxenic reference group, both groups are reared at about 25°C up to D7;
- on D7: at least 60 *Drosophila* larvae from each group are recovered and heat-shocked;
- the mean length of the larvae of each group is determined, and the means obtained are compared;
- said bacterial strain having, relative to the reference bacterium, a strong effect with a mean of the length of the larvae of the monoxenic group tested with this bacterial strain significantly higher than the mean for the monoxenic group with the reference strain, with a p-value less than or equal to 0.001, preferably to 0.0001 in the Mann-Whitney statistical test, carried out on the entire data set of the sizes of the larvae of both groups.

2. The composition for use as claimed in claim 1, wherein the *Lactobacillus* strain is selected from *L. plantarum* WJL, *L. plantarum* G821, and *L. fermentum* ATCC9338.

3. The composition for use as claimed in claim 1, comprising in said test A) a bacterial strain which, relative to the strain *L. plantarum* WJL, in the same *Drosophila* test, has a strong effect with a mean of the length of the larvae of the monoxenic group with this strain of bacteria significantly higher than the mean for the monoxenic group with the strain WJL, when the p-value is less than or equal to 0.001, preferably to 0.0001 in the Mann-Whitney statistical test, performed on the entire data set of the sizes of the larvae of both groups.

4. The composition for use as claimed in claim 1, comprising in said test B) a bacterial strain which, relative to the strain *L. plantarum* WJL, in the same mouse test, has a strong effect with a mean of the size of the mice or of their femurs of the monoxenic group with this bacterial strain significantly higher than the mean for the monoxenic group with the strain WJL, when the p-value is less than or equal to 0.05 in Tukey's statistical test.

5. The composition for use as claimed in claim 1, comprising in said test C) a bacterial strain which, relative to the strain *L. plantarum* WJL, in the same mouse test, has a strong effect with a mean serum IGF-1 level of the monoxenic group with this bacterial strain significantly higher than the mean for the monoxenic group with the strain WJL, when the p-value is less than or equal to 0.05 in Tukey's statistical test.

6. The composition for use as claimed in any one of claims 1 to 5, wherein the malnutrition in mammals is **characterized by** protein deficiency.

7. The composition for use as claimed in any one of the preceding claims, wherein the malnutrition in mammals is **characterized by** a below-normal serum IGF-1 level.

8. The composition for use as claimed in any one of the preceding claims, wherein the bacterium is a *Lactobacillus plantarum.*

9. The composition for use as claimed in claim 8, wherein the bacterium is a *Lactobacillus plantarum* G821 deposited at the *Collection Nationale de Culture de Microorganismes* (Institut Pasteur) under the registration number CNCM I-4979.

10. The composition for use as claimed in any one of the preceding claims, containing from $10^5$ to $10^{12}$ CFU of *Lactobacillus* responding positively to the test, per gram of composition.

11. A method for screening bacteria capable of promoting juvenile growth in case of malnutrition of mammals, in particular humans, livestock, pets and sporting animals, wherein the bacterial strain is subjected to the following test:

- a batch of embryos of axenic *Drosophila melanogaster* parents is available;
- these embryos are placed in at least 2 groups on a nutritional medium comprising a yeast-deficient nutrient medium for *Drosophila*, one of the embryo groups is further inoculated with a suspension of the bacterium to be tested, which forms the group of monoxenic individuals; the other group forms the axenic group,
- the rearing is conducted at a suitable temperature,
- at the end of a suitable rearing period, *Drosophila* larvae from each group (axenic and monoxenic larvae) are recovered, preferably in equal numbers,
- the mean length of the larvae from each group is determined, and the means obtained are compared;
- the tested bacterial strain is considered to respond positively to the test if the mean of the length of the larvae of the monoxenic group is greater than the mean of the length of the larvae of the axenic group with a p-value less than or equal to 0.001, preferably less than or equal to 0.0001 in the Mann-Whitney test, carried out on the entire data set.

12. A method for screening bacteria capable of promoting juvenile growth in the case of malnutrition, wherein the bacterial strain is subjected to the following test:

- juveniles are available from two lines of the same mouse strain, namely an axenic mouse line and a monoxenic mouse line,
- they are reared on a protein-deficient and also preferably lipid-deficient nutritional diet;
- at the end of a suitable rearing period, the mean of the values of one or more parameters of each group is determined, these parameters being related to the serum IGF-1 level or to growth,
- the lactic acid bacterium strain is considered to respond positively to the test if the mean IGF-1 level of the monoxenic group is higher than the mean level of the axenic group with a p-value less than or equal to 0.05 in Tukey's statistical test.

13. A composition, a pure culture or an isolate comprising the bacterium *Lactobacillus plantarum* G821 deposited at the *Collection Nationale de Culture de Microorganismes* (Institut Pasteur) under the registration number CNCM I-4979.

Fig.1

Fig.2

Fig.3

*Fig.4*

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **EUN-KYOUNG KIM et al.** *Genome Announcements,* Novembre 2013, vol. 1 (6), e0093713
- **JH RYU et al.** *Science,* 2008, vol. 319, 777-782
- **KORYSZEWSKA-BAGINSKA A. et al.** *Genome Announc,* 26 Septembre 2013
- **YUKI N et al.** *Int J Food Microbiol.,* 01 Avril 1999, vol. 48 (1), 51-7
- **ALEKSANDRZAK-PIEKARCZYK T. et al.** *Genome Announc,* 15 Août 2013
- **KORYSZEWSKA-BAGINSKA A. et al.** *Genome Announc,* 20 Février 2014
- **KANKAINEN M. et al.** *Proc Natl Acad Sci USA,* 06 Octobre 2009
- **G. STORELLI et al.** *Cell Metabolism,* 2011, vol. 14, 403-414
- **KIM et al.** *Genome Announc.,* 21 Novembre 2013, vol. 1 (6), e00937-13
- **MAZÉ et al.** *J. Bacteriol.,* Mai 2010, vol. 192 (10), 2647-8
- **VAN DE GUCHTE M et al.** *Proc.Natl Acad Sci USA,* 13 Juin 2006
- **KORYSZEWSKA-BAGINSKA A et al.** *Genome Announc,* 26 Septembre 2013
- **ALEKSANDRZAK-PIEKARCZYK T et al.** *Genome Announc,* 15 Août 2013
- **KORYSZEWSKA-BAGINSKA A et al.** *Genome Announc,* 20 Février 2014
- **KANKAINEN M et al.** *Proc Natl Acad Sci USA,* 06 Octobre 2009